# EUROPEAN PATENT APPLICATION

(11) **EP 4 721 734 A1**
(43) Date of publication of application: **08.04.2026**
(21) Application number: 24814573.2
(22) Date of filing: 31.05.2024
(51) Int. Cl.: A61K 31/136, A61K 31/7068, A61K 9/127, A61P 35/00

(54) **USE OF PHARMACEUTICAL COMPOSITION COMPRISING MITOXANTRONE LIPOSOME AND CYTARABINE IN THE TREATMENT OF ACUTE MYELOID LEUKEMIA**

(30) Priority: 02.06.2023 CN 202310648091
(71) Applicant: CSPC Zhongqi Pharmaceutical Technology (Shijiazhuang) Co., Ltd., Shijiazhuang, Hebei 050035 (CN)
(72) Inventor: LI, Chunlei, Shijiazhuang, Hebei 050035 (CN); LI, Yanhui, Shijiazhuang, Hebei 050035 (CN); HUANG, Wei, Shijiazhuang, Hebei 050035 (CN); LIU, Yanping, Shijiazhuang, Hebei 050035 (CN); LI, Mengmeng, Shijiazhuang, Hebei 050035 (CN); WANG, Caixia, Shijiazhuang, Hebei 050035 (CN); ZHOU, Meng, Shijiazhuang, Hebei 050035 (CN); WANG, Shixia, Shijiazhuang, Hebei 050035 (CN); YANG, Sensen, Shijiazhuang, Hebei 050035 (CN)
(74) Representative: Mewburn Ellis LLP
(86) International application number: PCT/CN2024/096549
(87) International publication number: WO 2024/245378

(57) **Abstract**

The use of mitoxantrone liposome combined with cytarabine, or further combined with other drugs, such as homoharringtonine or venetoclax, in the preparation of drugs used for treating acute myeloid leukemia (AML). Provided is a method of treating AML, the method comprising administering to an AML patient a pharmaceutical composition comprising mitoxantrone liposome and cytarabine, wherein the pharmaceutical composition may also comprise homoharringtonine or venetoclax.

## Description

### RELATED APPLICATION

This application claims priority to Chinese Patent Application No. 202310648091.9, filed on June 2, 2023, the entire contents of which are incorporated herein by reference for all purposes.

### TECHNICAL FIELD

The present application belongs to the field of pharmaceuticals, and particularly relates to the use of mitoxantrone liposome combined with cytarabine, or further combined with other drugs, in the treatment of acute myeloid leukemia (AML).

### BACKGROUND

Acute myeloid leukemia (AML) is a type of malignant proliferative disease originating from hematopoietic stem cells, and its biological characteristics are highly heterogeneous, with the highest incidence among acute leukemias in China. The incidence of leukemia in China is 5.17 per 100,000, and AML is the most common type of leukemia, accounting for 58.7% of all leukemias, and it is showing an increasing trend year by year. The incidence of AML increases with age, with a median age of onset of 68 years, and it is more common in men than women. AML has a rapid onset, rapid progress and poor prognosis. Although 60-80% of patients with initial treatment can achieve complete remission (CR) after induction therapy, unfortunately, most patients will relapse after entering remission, and only about less than 50% of patients can achieve a second remission. The 2-year and 5-year survival rates for AML patients are only 32% and 24%, making it a dangerous disease that seriously threatens human life and health.

Currently, although research on AML is mostly focused on targeted drugs, its treatment is still mainly chemotherapy. Over the past 35-40 years, multiple clinical research groups have explored different induction chemotherapy regimens. To date, cytarabine (Ara-C) combined with anthracyclines is still recognized both domestically and internationally as the best induction therapy regimen, and commonly used regimens include the DA regimen of cytarabine combined with daunorubicin (DNR), the IA regimen of cytarabine combined with idarubicin (IDA), and the MA regimen of cytarabine combined with mitoxantrone hydrochloride (MTO).

Liposomes are a novel form of drug delivery. Researchers have conducted studies on liposomal formulations of mitoxantrone. For example, mitoxantrone liposome is disclosed in Chinese patent application 200610102339.8, filed on December 29, 2006, and PCT application WO2008/080367A1, filed on December 29, 2007, the disclosures of which are incorporated herein by reference in their entirety.

Although topoisomerase II inhibitors of the same class have been proven safe, effective, and widely used in subjects with solid tumors and hematologic malignancies, data from studies on mitoxantrone hydrochloride liposome injection for the treatment of hematologic malignancies are limited.

Homoharringtonine (HHT), isolated from *Cephalotaxus* plants, has the effects of inhibiting protein synthesis, directly interfering with DNA polymerase activity, and inducing differentiation and apoptosis of leukemia cells. It is a cell cycle-specific drug that mainly acts on G1/G2 cells.

Bcl-2 is an important member of the anti-apoptotic protein subfamily, which has the function of inhibiting cell apoptosis and plays an important role in the occurrence and development of tumors. Venetoclax is the first oral bcl-2 inhibitor in the world with a high affinity and a unique mechanism of action that targets tumor cell apoptosis.

### SUMMARY OF THE INVENTION

The present application provides the use of a pharmaceutical composition comprising mitoxantrone liposome and cytarabine in the treatment of acute myeloid leukemia (AML). The inventors unexpectedly discovered during their research that the use of mitoxantrone liposome combined with cytarabine, or mitoxantrone liposome and cytarabine combined with other drugs such as homoharringtonine or venetoclax, has a good therapeutic effect on acute myeloid leukemia.

In a first aspect, the present application provides the use of a pharmaceutical composition comprising mitoxantrone liposome and cytarabine in the manufacture of a medicament for treating AML.

In some embodiments, the present application provides the use of mitoxantrone liposome and cytarabine in the manufacture of a medicament for treating AML.

In some embodiments, the present application provides the use of mitoxantrone liposome, cytarabine, and homoharringtonine in the manufacture of a medicament for treating AML.

In some embodiments, the present application provides the use of mitoxantrone liposome, cytarabine, and venetoclax in the manufacture of a medicament for treating AML.

In a second aspect, the present application provides a medicament or pharmaceutical composition for treating AML, comprising mitoxantrone liposome and cytarabine.

In some embodiments, the above medicament or pharmaceutical composition for treating AML further comprises homoharringtonine or venetoclax.

In a third aspect, the present application provides a pharmaceutical composition comprising mitoxantrone liposome and cytarabine, for use in the treatment of AML.

In some embodiments, the pharmaceutical composition further comprises other drugs, such as homoharringtonine or venetoclax.

In a fourth aspect, the present application provides a method of treating AML, comprising administering a therapeutically effective amount of mitoxantrone hydrochloride liposome and cytarabine hydrochloride to a patient or subject in need thereof.

In some embodiments, the method further comprises the combined use of homoharringtonine or venetoclax.

In a fifth aspect, the present application also provides a method of improving the therapeutic effect of homoharringtonine or venetoclax on an AML patient, comprising further administering a therapeutically effective amount of mitoxantrone liposome combined with cytarabine on the basis of administering homoharringtonine or venetoclax to the AML patient.

In a sixth aspect, the present application also provides the use of mitoxantrone liposome in the manufacture of a medicament for treating AML combined with cytarabine.

In some embodiments, the present application provides the use of mitoxantrone liposome in the manufacture of a medicament for treating AML combined with cytarabine and homoharringtonine.

In some embodiments, the present application provides the use of mitoxantrone liposome in the manufacture of a medicament for treating AML combined with cytarabine and venetoclax.

In a seventh aspect, the present application also provides a medicament or pharmaceutical composition for treating AML, comprising mitoxantrone liposome and a package insert or a drug label.

In some embodiments, the following contents are described in the package insert or the drug label:
(1) the mitoxantrone liposome is used combined with cytarabine for the treatment of AML; and/or
(2) the mitoxantrone liposome is used combined with cytarabine and homoharringtonine for the treatment of AML; and/or
(3) the mitoxantrone liposome is used combined with cytarabine and venetoclax for the treatment of AML.

In a preferred embodiment, the mitoxantrone liposome described in the above first to seventh aspects is mitoxantrone hydrochloride liposome.

In some embodiments, the AML described in the above first to seventh aspects is selected from newly diagnosed de novo AML or relapsed/refractory AML.

In some embodiments, the newly diagnosed de novo AML is selected from low-risk, intermediate-risk, or high-risk newly diagnosed de novo AML.

In some embodiments, the medicament or pharmaceutical composition described in the above first to seventh aspects can further comprise other first-line or second-line drugs for the treatment of AML, such as aclarubicin, decitabine, or azacitidine, etc. In some embodiments, the drugs refer to first-line or second-line drugs approved for the treatment of AML by drug regulatory authorities in China or other countries and regions (such as the United States, the European Union, Japan, or South Korea, etc.).

In some embodiments, in the medicament or pharmaceutical composition described in the above first to seventh aspects, different active ingredients can be comprised in the same formulation, forming a compound formulation product; or different active ingredients can be formulated separately into clinically acceptable dosage forms and combined and packaged into the product.

In a preferred embodiment, mitoxantrone, cytarabine, and homoharringtonine are injectable dosage forms, including liquid injections, injectable powders, injectable tablets, etc., and venetoclax is an oral dosage form. When the mitoxantrone hydrochloride liposome is a liquid injection, it comprises 0.5-5 mg/ml of active ingredient, preferably 1-2 mg/ml, more preferably 1 mg/ml, as calculated based on the mitoxantrone.

In some embodiments, mitoxantrone liposome, cytarabine, and homoharringtonine are preferably administered by injection, and venetoclax is preferably administered orally.

In some embodiments, the therapeutically effective amount of the mitoxantrone hydrochloride liposome is 8-36 mg/m², preferably 24-36 mg/m², as calculated based on the mitoxantrone. In a specific embodiment, the therapeutically effective amount can be a range between any two of the above doses, for example, 24 mg/m², 30 mg/m², and 36 mg/m².

In some embodiments, the mitoxantrone hydrochloride liposome is administered once every 4 weeks, and no more than 2 cycles at most.

In a preferred embodiment, the mitoxantrone hydrochloride liposome is administered intravenously each time, with the infusion time of the liposomal drug formulation being 30 min-120 min, preferably 60 min-120 min, for example 60 ± 15 min.

In some embodiments, the cytarabine described in the above first to seventh aspects is cytarabine hydrochloride. In some embodiments, the dose of the cytarabine hydrochloride is 100-1,000 mg/m³ as calculated based on the cytarabine, and preferably, the cytarabine hydrochloride is administered by intravenous infusion. The dosing cycle of the cytarabine hydrochloride is same as that of the mitoxantrone hydrochloride liposome.

In some embodiments, the homoharringtonine described in the above first to seventh aspects is formulated as a homoharringtonine injection. In some embodiments, the dose of the homoharringtonine injection is 2 mg/m², as calculated based on the homoharringtonine, and preferably, the homoharringtonine injection is administered by intravenous infusion. The dosing cycle of the homoharringtonine injection is same as that of the mitoxantrone hydrochloride liposome.

In some embodiments, the venetoclax described in the above first to seventh aspects is formulated as a venetoclax tablet. In some embodiments, the dose of the venetoclax tablet is 100-400 mg, as calculated based on the venetoclax, and preferably, the venetoclax tablet is administered orally. The dosing cycle of the venetoclax tablet is same as that of the mitoxantrone hydrochloride liposome.

In some embodiments, the present application provides a method of treating AML, with 4 weeks as a treatment phase. During the first treatment phase, mitoxantrone liposome injection is administrated at a dose of 24 mg/m², or 30 mg/m², or 36 mg/m² on day 1; and cytarabine is administrated at a dose of 100 mg/m² once daily from days 1-7.

In some embodiments, the present application provides a method of treating AML, with 4 weeks as a treatment phase. During the first treatment phase, mitoxantrone liposome injection is administrated at a dose of 24 mg/m² on day 1; 100 mg/m² cytarabine is administered once daily from days 1-4; 1 g/m² cytarabine is administrated every 12 hours on days 5, 6, and 7; and homoharringtonine is administrated at a dose of 2 mg/m² once daily from days 1-7.

In some embodiments, the present application provides a method of treating AML, with 4 weeks as a treatment phase. During the first treatment phase, administration of mitoxantrone liposome injection is administrated at a dose of 30 mg/m² on day 1; standard-dose cytarabine is administrated at a dose of 100 mg/m² once daily from days 1-7; 100 mg venetoclax is administrated on day 4, 200 mg venetoclax is administrated on day 5, and 400 mg venetoclax is administrated once daily from days 6-12.

In some embodiments, the above three methods of treating AML further comprise a second treatment phase: administration of mitoxantrone liposome injection at a dose of 24 mg/m², or 30 mg/m², or 36 mg/m² on day 1; administration of cytarabine at a dose of 100 mg/m² once daily from days 1-7; administration of 100 mg venetoclax on day 4, 200 mg venetoclax on day 5, and 400 mg venetoclax once daily from days 6-12.

The dosage of mitoxantrone hydrochloride liposome described in the present application is calculated based on the mitoxantrone.

The mitoxantrone hydrochloride liposome described in the present application can be prepared using conventional methods in the art, and can be that prepared by any method disclosed in the prior art, such as the method disclosed in WO2008/080367A1, the disclosure of which is incorporated herein by reference in their entirety.

In some embodiments, the medicament, pharmaceutical composition or mitoxantrone hydrochloride liposome described in the above first to seventh aspects has one or more of the following properties:
(i) the mitoxantrone hydrochloride liposome has a particle size of about 30-80 nm, for example about 35-75 nm, about 40-70 nm, about 40-60 nm or about 60 nm;
(ii) mitoxantrone hydrochloride forms an insoluble precipitate with multivalent counterions (such as sulfate, citrate or phosphate) in the liposome;
(iii) the phospholipid bilayer of the mitoxantrone hydrochloride liposome comprises phospholipid with a phase transition temperature (Tm) higher than body temperature, so that the phase transition temperature of the liposome is higher than body temperature, and for example, the phospholipid is selected from hydrogenated soybean lecithin, phosphatidylcholine, hydrogenated egg yolk lecithin, dispalmitoyl lecithin, distearoyl lecithin, or any combination thereof;
(iv) the phospholipid bilayer of the mitoxantrone hydrochloride liposome comprises hydrogenated soybean lecithin, cholesterol and polyethylene glycol 2000 modified distearoyl phosphatidylethanolamine (DSPE-PEG2000);
(v) the phospholipid bilayer of the mitoxantrone hydrochloride liposome comprises hydrogenated soybean lecithin, cholesterol and polyethylene glycol 2000 modified distearoyl phosphatidylethanolamine in a mass ratio of about 3:1:1, the mitoxantrone hydrochloride forms an insoluble precipitate with the polyvalent acid radical ions within the liposome, and the mitoxantrone hydrochloride liposome in the medicament has a particle size of about 60 nm; and
(vi) the mitoxantrone hydrochloride liposome is that with the National Drug Approval No. H20220001.

In some embodiments, the mitoxantrone hydrochloride liposome described herein has a particle size of about 30-80 nm, for example, any value between 30-80 nm. In some embodiments, the particle size is about 35-75 nm, preferably 40-70 nm, more preferably 40-60 nm, for example 60 nm.

In some embodiments, the mitoxantrone hydrochloride liposome described herein comprises mitoxantrone as an active ingredient and a phospholipid bilayer.

In some embodiments, mitoxantrone as an active ingredient can form an insoluble precipitate with the multivalent counterions in the liposome. In some embodiments, the counterion is sulfate, citrate, or phosphate.

In some embodiments, the above phospholipid bilayer comprises phospholipid with a phase transition temperature (Tm) higher than body temperature, thereby the phase transition temperature of the liposome is higher than body temperature. In some embodiments, the phospholipid with a Tm above body temperature is phosphatidylcholine, hydrogenated soybean lecithin, hydrogenated egg yolk lecithin, dispalmitoyl lecithin, distearoyl lecithin, or any combination thereof.

In some embodiments, the phospholipid bilayer comprises hydrogenated soybean lecithin, cholesterol, and polyethylene glycol 2000-modified distearoyl phosphatidylethanolamine. In some embodiments, the mass ratio of hydrogenated soybean lecithin, cholesterol, and polyethylene glycol 2000-modified distearoyl phosphatidylethanolamine in the phospholipid bilayer is 3:1:1.

In some embodiments, the mitoxantrone hydrochloride liposome described herein has a particle size of about 60 nm, and the counterion within the liposome is a sulfate ion.

In some embodiments, the phospholipid bilayer of the mitoxantrone hydrochloride liposome described herein comprises hydrogenated soybean lecithin, cholesterol, and polyethylene glycol 2000-modified distearoyl phosphatidylethanolamine in a mass ratio of 3:1:1, the liposome has a particle size of about 40-60 nm, and the counterion is a sulfate ion. In some embodiments, the weight ratio of HSPC:Chol:DSPE-PEG2000:mitoxantrone in the mitoxantrone hydrochloride liposome described herein is 9.58:3.19:3.19:1.

In the context of the present application, the preparation method of mitoxantrone liposome is as follows. HSPC (hydrogenated soybean lecithin), Chol (cholesterol) and DSPE-PEG2000 (polyethylene glycol 2000-modified distearoyl phosphatidylethanolamine) were weighed in a mass ratio of 3:1:1 and dissolved in 95% ethanol to obtain a clear solution. The ethanol solution of phospholipid was mixed with a 300 mM ammonium sulfate solution and hydrated by shaking at 60-65°C for 1h to obtain heterogeneous multi-compartment liposome. The particle size of the liposome was then reduced using a microfluidic device. The obtained sample was diluted 200 times with a 0.9% NaCl solution and then detected by NanoZS, the average particle size of the particles was about 60 nm, and the main peak was concentrated between 40 and 60 nm. Then, the ammonium sulfate in the outer phase of the blank liposome was removed by an ultrafiltration device, and the external phase was replaced with 290 mM sucrose and 10 mM glycine to form a transmembrane ammonium sulfate gradient. A mitoxantrone hydrochloride solution (10 mg/mL) was added to the blank liposome at a lipid-to-drug ratio of 16:1, and the drug was loaded at 60-65°C. After incubation for about 1 hour, gel exclusion chromatography can demonstrate that the encapsulation efficiency was approximately 100%. The weight ratio of HSPC:Chol:DSPE-PEG2000:mitoxantrone was 9.58:3.19:3.19:1, and the osmotic pressure of the sucrose-glycine solution was close to the physiological value.

It should be understood that many of the technical details and parameters in the above exemplary preparation method can be adjusted and determined by those skilled in the art within a reasonable range. For example, the amino acids that can be replaced by glycine in the external phase used to form the transmembrane ammonium sulfate gradient include, but are not limited to, histidine, asparagine, glutamic acid, leucine, proline, and alanine. For example, the mass ratio of HSPC, Chol, and DSPE-PEG2000 can be adjusted appropriately. For example, regarding the lipid-to-drug ratio in the preparation of a specific liposomal drug formulation, those skilled in the art can design, test, and ultimately obtain a suitable lipid-to-drug ratio to maximize drug loading while minimizing drug leakage, and for the mitoxantrone hydrochloride liposome formulation of the present application, a wide range of lipid-to-drug ratios can be used, for example, as low as 2:1 or as high as 30:1, 40:1, or 50:1, and a more suitable lipid-to-drug ratio can be about (15-20):1, such as about 15:1, 16:1, 17:1, 18:1, 19:1, or 20:1. Therefore, the several advantageous properties of the mitoxantrone hydrochloride liposome formulation described above are more important, and there are various methodologies for achieving these properties.

### DETAILED DESCRIPTION

The present application provides a pharmaceutical composition for effectively treating AML, which comprises mitoxantrone liposome and cytarabine, and can also comprise other drugs such as homoharringtonine or venetoclax.

In some embodiments, combined administration of 1) mitoxantrone hydrochloride liposome and cytarabine hydrochloride; or 2) mitoxantrone hydrochloride liposome, cytarabine hydrochloride and homoharringtonine; or 3) mitoxantrone hydrochloride liposome, cytarabine hydrochloride and venetoclax to an AML patient can improve the therapeutic effect of cytarabine alone or in combination with other drugs on a patient with de novo newly diagnosed AML or relapsed/refractory AML, increase the disease remission rate, and is safe, well-tolerated, and has few side effects. Especially, the combined administration can improve the complete remission rate (CR rate) and partial remission rate (PR rate) of the disease, and can control disease progression, and prolong event-free survival (EFS) and relapse-free survival (RFS).

The pharmaceutical composition provided in the present application can be applied to the field of anti-tumor therapy, and is particularly suitable for the treatment of AML.

Unless otherwise specified, the terms used in the present application are defined as follows:
AML stands for acute myeloid leukemia. "Newly diagnosed" is defined as being diagnosed for the first time and not yet having received anti-leukemia therapy.

"Low risk" is defined as being classified as low risk according to the prognostic stratification criteria of "Revised 2022 European LeukemiaNet (ELN) Recommendations for Diagnosis and Management of AML in Adults".

"Intermediate risk" is defined as being classified as intermediate risk according to the prognostic stratification criteria of "Revised 2022 European LeukemiaNet (ELN) Recommendations for Diagnosis and Management of AML in Adults".

"High risk" is defined as being classified as high risk according to the prognostic stratification criteria of "Revised 2022 European LeukemiaNet (ELN) Recommendations for Diagnosis and Management of AML in Adults".

"De novo" is defined as leukemia that is not secondary to other hematological diseases or radiotherapy/chemotherapy.

"Relapse" is defined as the reappearance of leukemia cells in the peripheral blood or ≥5% of primitive cells in the bone marrow (excluding other causes such as bone marrow regeneration after consolidation chemotherapy) or leukemia cell infiltration outside the marrow after complete remission (CR).

"Refractory" is defined as: 1) newly diagnosed cases showing no response after two courses of standard treatment; 2) cases that relapse within 12 months following consolidation therapy after complete remission (CR); 3) cases that relapse after 12 months but do not respond to conventional chemotherapy; 4) cases that relapse twice or more; or 5) cases with persistent extramedullary leukemia.

The CR/CRh rate is defined as the proportion of subjects whose best response is complete remission (CR) and complete remission with partial hematological recovery (CRh) in the analyzed population.

The CRc rate is defined as the proportion of subjects whose best response is complete remission (CR), complete remission with partial hematological recovery (CRh), and complete remission with incomplete hematological recovery (CRi) in the analyzed population.

In this specification and claims, the terms "comprise/comprising," "include/including," and "contain/containing" mean "including but not limited to" and are not intended to exclude other parts, additives, components, or steps.

It should be understood that the features, characteristics, components or steps described in a particular aspect, embodiment or example of this application can be applied to any other aspect, embodiment or example described herein, unless contradicted by context.

The above disclosure generally describes the present invention, which is further exemplified by the following examples. These examples are described merely to illustrate the invention and are not intended to limit the scope of the invention. Although specific terms and values are used herein, they are also to be understood as exemplary and do not limit the scope of the invention. Unless otherwise specified, the experimental methods and techniques in this specification are well known to those skilled in the art. Unless otherwise stated, the raw materials and reagents used in the following examples are commercially available products or can be prepared by known methods.

### Example 1: A clinical study evaluating the tolerability and efficacy of mitoxantrone hydrochloride liposome injection combined with cytarabine in patients with acute myeloid leukemia.

### 1. Objectives of the experiment

Primary objective: To evaluate the tolerability of different doses of mitoxantrone hydrochloride liposome injection combined with cytarabine in relapsed/refractory acute myeloid leukemia (R/R AML).

Secondary objective: To evaluate the safety and efficacy of mitoxantrone hydrochloride liposome injection combined with cytarabine in R/R AML.

### 2. Experimental design: A single-arm, non-randomized, open-label experiment.

### 3. Inclusion criteria

1) Subjects participated in the research voluntarily and signed informed consents;
2) Subjects should be 18 years of age or older, both men and women being eligible;
3) Subjects were newly diagnosed or relapsed/refractory AML subjects whose morphological and/or pathological diagnosis met the World Health Organization (WHO) 2016 classification criteria for AML diagnosis;
4) ECOG score of the subjects was 0-2;
5) Organ function levels of the subjects must meet the following requirements: (1) liver: alanine aminotransferase (AST)/aspartate aminotransferase (ALT) ≤ 3 x the upper limit of normal (ULN); total bilirubin ≤ 1.5 x ULN; (2) kidney: serum creatinine ≤ 1.5 x ULN; and
6) Subjects and their partners shall take effective contraceptive measures during the trial period and within 6 months after the last dose; and female subjects should be negative for HCG in urine or blood (excluding those with menopause and hysterectomy).

### 4. Exclusion criteria

1) AML presented with any of the following conditions: (1) acute promyelocytic leukemia; (2) AML with acute transformation of chronic myeloid leukemia; or (3) central nervous system leukemia.
2) Subjects had other malignant tumors in the past 5 years (excluding cured basal cell carcinoma of the skin and cervical carcinoma in situ).
3) Subjects had graft-versus-host disease requiring continuous treatment, or had received more than one autologous or allogeneic stem cell transplant.
4) Subjects had allergic history of mitoxantrone hydrochloride injection, cytarabine, or liposomal preparations.
5) Subjects had previously been treated with doxorubicin or other anthracyclines, and the cumulative dose of doxorubicin exceeded 400 mg/m² (dose calculation equivalent to anthracycline: 1 mg doxorubicin = 2 mg epirubicin = 2 mg daunorubicin = 0.5 mg idarubicin = 0.45 mg mitoxantrone; except for doxorubicin liposomes).
6) Subjects received any anti-tumor treatment within 2 weeks prior to the first administration (or within 5 half-lives of the drug), including chemotherapy, immunotherapy, targeted therapy, endocrine therapy, or radiotherapy (with local radiotherapy intervals of < 2 weeks). Leukocytoreductive therapy (hydroxyurea, or leukapheresis, etc.) and prophylactic intrathecal injections administered more than 24 hours were excluded.
7) Patients were receiving systemic anti-infection treatment but had poor infection control (those who showed signs of infection progression within 1 week prior to the first administration, or as determined by the investigators).
8) Patients were receiving systemic anti-infection treatment but had poor infection control (those who showed signs of infection progression within 1 week prior to the first administration, or as determined by the investigators).
9) Subjects had other conditions judged by the investigators as unsuitable for participating in the study.

### 5. Test drugs

### 1) Chinese generic name: Mitoxantrone Hydrochloride Liposome Injection

### Specification: 10mg/10ml/vial

Usage and Dosage: Mitoxantrone hydrochloride liposome injection combined with cytarabine was used for treatment. Three dosage groups are set: 24 mg/m², 30 mg/m², and 36 mg/m²., and the dose for the dose expansion phase was determined based on the experimental results in the previous phase. The drug was administered once every 4 weeks (q4w), and on day 1 of each cycle (D1).

Duration of Administration: Maximum 2 cycles of administration (8 weeks).

### 2) Chinese generic name: Cytarabine Hydrochloride for Injection

### Specifications: 0.1g/vial, or 0.3g/vial

Usage and Dosage: Mitoxantrone hydrochloride liposome injection combined with cytarabine was used for treatment, and in the relapse/refractory group, the dosage is 1.5 g/m² per administration, administered every 12 hours (q12h) on Days 1, 3, and 5 of each cycle, with one cycle every 4 weeks.

Duration of Administration: Maximum 2 cycles of administration (8 weeks).

### Research Results

At present, the observation of dose-limiting toxicity (DLT) events of all subjects in the three dose groups had been completed, and a total of 14 subjects were enrolled in the 24, 30, and 36 mg/m² dose groups, with at least one efficacy evaluation in 13 subjects. All subjects had used anthracycline in the past, and the overall CR/CRh rate was 23.1%, and the CRc rate was 38.5%. Preliminary results showed that mitoxantrone liposome combined with cytarabine can produce therapeutic effects in R/R AML.

Seven subjects were enrolled in the 36 mg/m² dose group, and one of the seven subjects developed DLT, and the maximum tolerated dose (MTD) was not found.

### Example 2: A clinical study evaluating the safety, efficacy, and pharmacokinetics of combination therapy based on mitoxantrone hydrochloride liposome injection in the treatment of newly diagnosed de novo acute myeloid leukemia.

This study was an open-label, multi-cohort, multi-center clinical study that included newly diagnosed de novo AML subjects, and three cohort studies were carried out. Cohort 1 is the mitoxantrone hydrochloride liposome plus standard-dose cytarabine group; cohort 2 is the mitoxantrone hydrochloride liposome plus medium-dose cytarabine and homoharringtonine group; and cohort 3 is the mitoxantrone hydrochloride liposome plus standard-dose cytarabine and venetoclax group. The objectives of this study are to explore the safety and tolerability of these combination regimens, evaluate their efficacy and observe the pharmacokinetic characteristics of mitoxantrone liposome.

### I. Experimental Design

### (1) Overall experimental design

The study included a screening period, a treatment period, and a follow-up period.

After signing the informed consent form and completing all baseline examinations during the screening period, qualified subjects would enter the treatment period and be assigned to three different cohorts in a 1:1:1 ratio. The investigational intervention in this study was induction-phase therapy. All of the cohorts underwent a maximum of two cycles of induction therapy, with each cycle lasting 28 days. If the efficacy evaluation of a subject achieved CRc (CR/CRi/CRh) after the first cycle of induction therapy, he/she would discontinue treatment and entered the follow-up period for RFS, EFS, and OS follow-up. If a subject did not achieve the morphological leukemia-free state (MLFS) after the first cycle of induction therapy, a second cycle of re-induction therapy would be performed. The subjects in all cohorts chose the medication regime of cohort 3 for the second induction treatment.

### (2) Dosing Regimens for the first and second cycles

### Induction dosing regimen for the first cycle

Cohort 1: Mitoxantrone hydrochloride liposome injection was administered at a dose of 30 mg/m² on day 1; standard-dose cytarabine was administered at a dose of 100 mg/m² once daily from days 1-7. If less than 2 dose-limiting toxicity (DLT) events occurred in the first 6 subjects during the safety introduction period, the dose of mitoxantrone hydrochloride liposome can be increased to 36 mg/m².

Cohort 2: Mitoxantrone hydrochloride liposome injection was administered at a dose of 24 mg/m² on day 1; cytarabine at a dose of 100 mg/m² was administered once daily from days 1-4; cytarabine at a dose of 1 g/m² was administered once every 12 hours on days 5, 6, and 7; homoharringtonine was administered at a dose of 2 mg/m² once daily from days 1-7.

Cohort 3: Mitoxantrone hydrochloride liposome injection was administered at a dose of 30 mg/m² on day 1; cytarabine was administered at a dose of 100 mg/m² once daily from days 1-7 of each cycle; 100 mg venetoclax was administered on day 4, 200 mg venetoclax was administered on day 5, and 400 mg venetoclax was administered once daily from days 6-12.

### Induction dosing regimen for the second cycle

Mitoxantrone hydrochloride liposome injection was administered at a dose of 30 mg/m² on day 1; cytarabine was administered at a dose of 100 mg/m² once daily from days 1-7 of each cycle; 100 mg venetoclax was administered on day 4, 200 mg venetoclax was administered on day 5, and 400 mg venetoclax was administered once daily on days 6-12.

### II. Trial Population

### (I) Inclusion criteria

Subjects who met all of the following criteria were eligible to be included in this study:
1. Voluntarily participating in the research and signing the informed consent form;
2. Age ≥ 18 years and <65 years;
3. Newly diagnosed AML with ≥20% blasts based on morphology, conforming to the World Health Organization (WHO) 2016 classification criteria;
4. ECOG score of 0-1;
5. Judged by the investigator to be able to tolerate intensive chemotherapy;
6. Organ function levels must meet the following requirements:
   (1) liver: alanine aminotransferase (AST) or aspartate aminotransferase (ALT) ≤ 3 x the upper limit of normal (ULN); total bilirubin ≤ 1.5 x ULN;
   (2) kidney: serum creatinine ≤ 1.5 x ULN; and
7. Female/male subjects of childbearing potential must take adequate non-pharmacological contraception from the time of signing the informed consent form until 6 months after the last dose, and must not donate sperm or ova; the female subjects of childbearing potential must be negative for HCG in blood; and the female subjects of childbearing potential refer to the females excluding those who are postmenopausal (defined as no menstrual periods for at least 1 year) or have undergone sterilization surgery at least 1 month prior to screening.

### (II) Exclusion criteria

Subjects who met any of the following criteria were ineligible for inclusion in this study:
1. Diagnosed with any of the following conditions in the screening period:
   1) acute promyelocytic leukemia; or
   2) AML in blast crisis of chronic myeloid leukemia;
2. Subjects who had central nervous system leukemia;
3. Subjects who had AML secondary to radiotherapy or chemotherapy for other tumors;
4. Subjects who had other malignant tumors in the past 5 years (excluding curatively treated cutaneous basal cell carcinoma and carcinoma in situ of the cervix);
5. Subjects who had already received anthracycline pretreatment or other anti-AML treatment (excluding leukocytoreductive therapy, such as hydroxyurea, or leukapheresis, etc.);
6. Subjects with a known history of immediate or delayed hypersensitivity to drugs of the same class as the investigational drug in this study;
7. Subjects with active infection or poorly controlled infection (with signs of infection progression within 1 week prior to the first administration, or as judged by the investigators);
8. Subjects with a history of severe hemorrhagic diseases, such as hemophilia A, hemophilia B, von Willebrand disease, or spontaneous bleeding requiring blood transfusions or other medical interventions;
9. Subjects whose expected survival was less than 3 months;
10. Subjects with an abnormality in the cardiac system; or
11. Subjects with other conditions judged by the investigators as unsuitable for participating in the study.

### (III) Treatment discontinuation criteria

Subjects must discontinue treatment with the investigational drug if any of the following conditions occurred during the study.
1. Subjects experienced intolerable adverse events;
2. Subjects completed the study treatment;
3. Subjects had poor compliance;
4. A major protocol deviation in the course of experiment prevented the experiment from proceeding smoothly;
5. Subjects began new anti-tumor treatment; or
6. Subjects were pregnant;

The subjects met any of the criteria for withdrawal from the study.

### (IV) Criteria for study withdrawal

Subjects had the right to withdraw from the study at any time for any reason. The subjects would withdraw from the study if any of the following conditions occurred:
1. Death;
2. Loss to follow-up;
3. Subjects were unwilling to continue to participate in the trial;
4. The sponsor decided to terminate the trial in advance; or
5.Other reasons.

### (V) Evaluation criteria

The efficacy evaluation criteria were based on "Revised 2022 European LeukemiaNet (ELN) Recommendations for Diagnosis and Management of AML in Adults".

### III. Research Results

Mitoxantrone hydrochloride liposome combined with cytarabine, or mitoxantrone hydrochloride liposome combined with cytarabine and homoharringtonine, or mitoxantrone hydrochloride liposome combined with cytarabine and venetoclax were safe and well-tolerated, with few side effects and significant efficacy in both newly diagnosed and relapsed/refractory AML patients, and particularly, the CR rates of different subjects for the diseases were improved.

As of May 10, 2024, a total of 7 subjects in cohort 1 (mitoxantrone hydrochloride liposome combined with cytarabine) underwent efficacy evaluation (as for the dosage of mitoxantrone hydrochloride, 6 subjects received 30 mg/m² and 1 subject received 36 mg/m²), and the CR rate was 57.1%, the CRc rate was 71.4%, and the minimal residual disease (MRD) negative rate was 57.1%.

In cohort 2 (mitoxantrone hydrochloride liposome combined with cytarabine and homoharringtonine), a total of 7 subjects underwent efficacy evaluation, and the CR rate was 71.4%, the CRc rate was 86.7%, and the MRD negative rate was 42.9%.

In cohort 3 (mitoxantrone hydrochloride liposome combined with cytarabine and venetoclax), a total of 8 subjects underwent efficacy evaluation, and the CR rate was 62.5%, the CRc rate was 100%, and the MRD negative rate was 50%.

The results can also be found in Table 1 below.

**Table 1**

| | | Cohort 1 | | Cohort 2 | | Cohort 3 | | In total |
|---|---|---|---|---|---|---|---|---|
| | | N=7 | | N=7 | | N=8 | | N=22 |
| CR rate | 57.1% (4/7) | | 71.4% (5/7) | | 62.5% (5/8) | | 63.6% (14/22) | |
| CRc rate | 71.4% (5/7) | | 85.7% (6/7) | | 100% (8/8) | | 86.4% (19/22) | |

The above experimental results showed that mitoxantrone hydrochloride liposome combined with cytarabine, or mitoxantrone hydrochloride liposome combined with cytarabine and homoharringtonine, or mitoxantrone hydrochloride liposome combined with cytarabine and venetoclax can effectively treat AML and has a broad clinical application prospect in the treatment of AML.

The implementations of the technical solutions of the present application have been illustratively described above. It is to be understood that the protection scope of the present application should not be unduly limited to these specific embodiments. Any modifications, equivalents, and improvements, etc. made by those skilled in the art within the spirit and principles of this application are intended to be included within the protection scope of the claims of this application.

## Claims

1. Use of mitoxantrone liposome and cytarabine in the manufacture of a medicament for treating acute myeloid leukemia (AML).

2. Use of mitoxantrone liposome, cytarabine and homoharringtonine in the manufacture of a medicament for treating AML.

3. Use of mitoxantrone liposome, cytarabine and venetoclax in the manufacture of a medicament for treating AML.

4. A pharmaceutical composition for treating AML, comprising mitoxantrone liposome and cytarabine.

5. The pharmaceutical composition according to claim 4, wherein the pharmaceutical composition further comprises homoharringtonine or venetoclax.

6. A pharmaceutical composition comprising mitoxantrone liposome and cytarabine, for use in the treatment of AML, wherein optionally, the pharmaceutical composition further comprises homoharringtonine or venetoclax.

7. A method for treating AML, which comprises administering a therapeutically effective amount of mitoxantrone liposome and cytarabine to a patient or subject in need thereof.

8. The method according to claim 7, wherein the method further comprises co-administering homoharringtonine or venetoclax.

9. A method for improving the therapeutic effect of homoharringtonine or venetoclax against AML, which comprises further administering a therapeutically effective amount of mitoxantrone liposome combined with cytarabine on the basis of administering homoharringtonine or venetoclax to the AML patient.

10. The method according to any one of claims 7-9, wherein the therapeutically effective amount of mitoxantrone liposome is 8-36 mg/m², preferably 24-36 mg/m², for example, 24 mg/m², 30 mg/ m², and 36 mg/ m², as calculated based on the mitoxantrone,
preferably, the mitoxantrone liposome is administered once every 4 weeks, and no more than 2 cycles,
preferably, the mitoxantrone liposome is administered intravenously each time, and the infusion time of the mitoxantrone liposome is 30 min-120 min, preferably 60 min-120 min; and/or
the cytarabine is formulated as cytarabine hydrochloride, and wherein the dose of the cytarabine hydrochloride is 100-1,000 mg/m³, as calculated based on the cytarabine, and preferably, the cytarabine hydrochloride is administered by intravenous infusion; and/or
the homoharringtonine is formulated as a homoharringtonine injection, and wherein the dose of the homoharringtonine injection is 2 mg/m², as calculated based on the homoharringtonine, and preferably, the homoharringtonine injection is administered by intravenous infusion; and/or
the venetoclax is formulated as a venetoclax tablet, wherein the dose of the venetoclax tablet is 100-400 mg, as calculated based on the venetoclax, and preferably, the venetoclax tablet is administered orally.

11. The method according to claim 7, wherein mitoxantrone liposome injection is administered at a dose of 30 mg/m² or 36 mg/m² on day 1, and cytarabine is administered at a dose of 100 mg/m² once daily from days 1-7.

12. The method according to claim 8, wherein mitoxantrone liposome injection is administered at a dose of 24 mg/m² on day 1, 100 mg/m² cytarabine is administered once daily from days 1-4; 1 g/m² cytarabine is administered every 12 hours on days 5, 6 and 7; and homoharringtonine is administered at a dose of 2 mg/ m² once daily from days 1-7.

13. The method according to claim 8, wherein mitoxantrone liposome injection is administered at a dose of 30 mg/m² on day 1, standard-dose cytarabine is administered at a dose of 100 mg/m² once daily from days 1-7, 100 mg venetoclax is administered on day 4, 200 mg venetoclax is administered on day 5, and 400 mg venetoclax is administered once daily from days 6-12.

14. The method according to any one of claims 11-13, which further comprises a next phase of treatment: administration of mitoxantrone liposome injection at a dose of 30 mg/m² on day 1; administration of cytarabine at a dose of 100 mg/m² once daily from days 1-7; administration of 100 mg venetoclax on day 4, administration of 200 mg venetoclax on day 5, and administration of 400 mg venetoclax once daily from days 6-12.

15. Use of mitoxantrone liposome in the manufacture of a medicament for treating AML combined with cytarabine.

16. Use of mitoxantrone liposome in the manufacture of a medicament for treating AML combined with cytarabine and homoharringtonine.

17. Use of mitoxantrone liposome in the manufacture of a medicament for treating AML combined with cytarabine and venetoclax.

18. A medicament or pharmaceutical composition for treating AML, which comprises mitoxantrone liposome, and a package insert or a drug label.

19. The medicament or pharmaceutical composition according to claim 18, wherein the following contents are described in the package insert or the drug label:
(1) the mitoxantrone liposome is used combined with cytarabine for the treatment of AML; and/or
(2) the mitoxantrone liposome is used combined with cytarabine and homoharringtonine for the treatment of AML; and/or
(3) the mitoxantrone liposome is used combined with cytarabine and venetoclax for the treatment of AML.

20. The use according to any one of claims 1-3 and 15-17, the pharmaceutical composition according to any one of claims 4-6, the method according to any one of claims 7-13, or the medicament or pharmaceutical composition according to claim 18 or 19, wherein the AML is selected from de novo AML, newly diagnosed AML or relapsed/refractory AML.

21. The use according to any one of claims 1-3 and 15-17, the pharmaceutical composition according to any one of claims 4-6, the method according to any one of claims 7-13, or the medicament or pharmaceutical composition according to claim 18 or 19, wherein the mitoxantrone liposome is mitoxantrone hydrochloride liposome, which has one or more of the following properties:
(i) the mitoxantrone hydrochloride liposome has a particle size of about 30-80 nm, for example about 35-75 nm, about 40-70 nm, about 40-60 nm, such as about 60 nm;
(ii) mitoxantrone hydrochloride in the mitoxantrone hydrochloride liposome forms an insoluble precipitate with multivalent counterions in the liposome, and the multivalent counterions are sulfate, citrate or phosphate, for example;
(iii) the phospholipid bilayer of the mitoxantrone hydrochloride liposome comprises phospholipid with a phase transition temperature (Tm) higher than body temperature, so that the phase transition temperature of the liposome is higher than body temperature, and for example, the phospholipid is selected from hydrogenated soybean lecithin, phosphatidylcholine, hydrogenated egg yolk lecithin, dipalmitoyl lecithin, distearoyl lecithin, or any combination thereof; and
(iv) the phospholipid bilayer of the mitoxantrone hydrochloride liposome comprises hydrogenated soybean lecithin, cholesterol and polyethylene glycol 2000 modified distearoyl phosphatidylethanolamine (DSPE-PEG2000);
preferably, the mass ratio of the hydrogenated soybean lecithin, cholesterol and polyethylene glycol 2000 modified distearoyl phosphatidylethanolamine is 3:1:1, and/or the mitoxantrone hydrochloride forms an insoluble precipitate with the polyvalent acid radical ions within the liposome, and/or a particle size of the mitoxantrone hydrochloride liposome in the medicament is about 60 nm.
